# FASCICULE DE BREVET EUROPEEN

(11) **EP 2 882 841 B1**
(45) Date de publication et mention de la délivrance du brevet: **27.12.2017**
(21) Numéro de dépôt: 13758947.9
(22) Date de dépôt: 07.08.2013
(51) Int. Cl.: C12N 1/12, C07C 403/24

(54) **PROCÉDÉ D'OBTENTION D'UNE COMPOSITION RICHE EN LUTÉINE PRODUITE PAR DES MICROALGUES**
VERFAHREN ZUR HERSTELLUNG EINER ZUSAMMENSETZUNG REICH AN LUTEIN AUS MIKROALGEN
METHOD FOR PREPARING A COMPOSITION RICH IN LUTEIN PRODUCED BY MICROALGAE

(30) Priorité: 08.08.2012 FR 1257691
(43) Date de publication de la demande: 17.06.2015
(73) Titulaire: Roquette Frères, 62136 Lestrem (FR)
(72) Inventeur: LOOTEN, Philippe, 59160 Lomme (FR); PATINIER, Samuel, 59000 Lille (FR); FRANCAIS, Eric, 54134 Voinemont (FR); PERRUT, Michel, 54600 Villers Les Nancy (FR); SATRE-BUISSON, Aurore, 54250 Champigneulles (FR)
(74) Mandataire: Verhaeghe Bect, Elodie
(86) Numéro de dépôt international: PCT/FR2013/051902
(87) Numéro de publication internationale: WO 2014/023917

(56) Documents cités:
- EP-A1- 0 761 765
- EP-A1- 1 808 483
- WO-A1-2004/094350
- WO-A1-2012/064186
- FR-A1- 2 924 126

## Description

### DOMAINE DE L'INVENTION

La présente invention est relative à un procédé d'obtention d'une composition riche en lutéine produite par des microalgues, plus particulièrement des microalgues de la famille des Chlorelles, plus particulièrement encore produite par *Chlorella vulgaris.*

### PRESENTATION DE L'ETAT DE LA TECHNIQUE

Les caroténoïdes sont des pigments plutôt oranges ou jaunes répandus chez de très nombreux organismes vivants. Liposolubles, ils sont en général facilement assimilables par les organismes.

Ils appartiennent à la famille chimique des terpénoïdes, formés à partir de la polymérisation d'unités isoprènes à structure aliphatique ou alicyclique. Il est généralement admis qu'ils suivent des voies métaboliques similaires à celles des lipides.

Ils sont synthétisés par toutes les plantes vertes et par de nombreux champignons, bactéries (dont les cyanobactéries) et par toutes les algues. Ils sont absorbés par les animaux dans leur nourriture.

Les caroténoïdes ont des propriétés antioxydantes et ont été étudiés pour la prévention du cancer et d'autres maladies humaines.

Les caroténoïdes sont naturellement présents dans les feuilles comestibles, les fleurs et les fruits, et sont facilement obtenus à partir des fleurs (par exemple, les tagètes), les baies, et les tissus racinaires (par exemple, les carottes).

Des exemples représentatifs de caroténoïdes comprennent l'α-carotène, le β-carotène et le lycopène.

Le β-carotène et le lycopène, sont généralement présents sous une forme non combinée libre, qui est emprisonnée au sein des chloroplastes des cellules végétales.

Les xanthophylles sont des molécules de couleur jaune dérivées des carotènes, par ajout d'atomes d'oxygène (fonctions alcool, cétone, époxy, ...).

Les xanthophylles sont abondantes dans un certain nombre de fruits jaunes ou oranges et légumes tels que les pêches, les mangues, la papaye, les pruneaux, les courges et les oranges.

On les rencontre également dans les chloroplastes ou les chromoplastes des cellules végétales, notamment dans les pétales de certaines fleurs de couleur jaune, orange ou rouge, et chez les algues, par exemple les algues brunes (Phéophycées), où elles masquent la chlorophylle.

Les xanthophylles sont des antioxydants qui contribuent, entre autres, à la santé des yeux.

Des exemples de xanthophylles comprennent la lutéine, l'astaxanthine, la canthaxanthine, la zéaxanthine, la cryptoxanthine...

Certaines xanthophylles sont présentes dans les fleurs de plantes, comme les tagètes, généralement sous la forme de diesters d'acides tels que les acides palmitique et myristique.

En règle générale, les formes libres de caroténoïdes sont également présentes dans les chloroplastes des plantes vertes telles que la luzerne, les épinards, le chou frisé, les feuilles et les matières végétales vertes.

La forme libre des caroténoïdes permet une meilleure absorption lorsqu'ils sont consommés dans les aliments ou comme complément alimentaire.

La lutéine est un pigment xanthophylle de formule 4[18-(4-hydroxy-2,6,6-triméthylcyclohex-1-én-I-yl)-3,7,12,16-tetraméthyloctadéca-1,3,5,7,9,11,13,15,17-nonaén-l-yl]-3,5,5-triméthylcyclohex-2-én-l-ol trouvé en concentrations élevées dans la macula de l'oeil et dans la partie centrale de la rétine.

Elle y joue un rôle important dans la filtration des longueurs d'onde ultraviolettes de la lumière afin d'éviter d'endommager la lentille de l'oeil et la macula.

La lutéine a par ailleurs des propriétés antioxydantes qui permettent également de protéger la macula, qui est riche en acides gras polyinsaturés, des radicaux libres induits par la lumière.

La lutéine ne peut pas être produite par le corps et, par conséquent, doit être apportée par l'alimentation.

Ainsi, la lutéine est devenue de plus en plus utilisée dans les compléments nutritionnels pour la prévention et / ou le traitement des pertes de vision dues à la dégénérescence maculaire liée à l'âge (ou DMLA), les cataractes, et la rétinite pigmentaire.

La source la plus commune d'extraits de lutéine est justement la fleur du souci, qui contient l'un des plus hauts niveaux de lutéine connus et qui présente l'avantage de ne contenir qu'une faible concentration des autres caroténoïdes.

La lutéine sous forme cristalline est classiquement obtenue à partir des fleurs de d'oeillet d'Inde (*Tagetes erecta*) de la famille des *Asteraceae* par extraction au moyen de solvants, afin de produire une oléorésine (à 70 % de lutéine).

Cette oléorésine est ensuite purifiée en procédant à d'autres séries d'extractions, soit au moyen de solvants (hexane, pentane, dichlorométhane, alcool éthylique, méthanol) ou en utilisant du 1,2-propylène glycol et du chlorhydrate de potassium.

Ce mode d'extraction en deux étapes est par exemple décrit dans la demande de brevet internationale WO 2012/064186.

Ces deux procédés mènent à un produit final dont 99 à 99,9 % des solvants organiques d'extraction ont été éliminés.

La lutéine cristalline peut être incorporée dans une suspension d'huile de maïs avant d'être commercialisée.

Les méthodes de purification de la lutéine, sous forme d'esters d'acides gras à partir de pétales de fleurs de tagètes, sont par exemple enseignées dans la littérature par les brevets US 4,048,203, US 5,382,714 et US 5,648,564, dans lesquels les pétales de fleurs de tagètes séchées sont traitées avec un solvant hydrocarboné.

Quant à l'extraction de lutéine des plantes vertes, elle peut être avantageuse, car elle ne nécessite pas d'étape chimique supplémentaire de saponification puis de lyse afin de libérer la lutéine sous forme libre, forme désirée pour une meilleure absorption lorsqu'elle est consommée.

Toutefois, l'extraction et la purification de la lutéine, mais également des carotènes, et des acides gras à partir de plantes n'est pas très économique parce qu'elle nécessite de nombreuses étapes de purification coûteuses et beaucoup de temps afin de les séparer des grandes quantités d'autres composés présents dans les matières végétales.

Par ailleurs, le contenu en lutéine des fleurs de tagètes reste faible (0,3 mg/g de matière sèche.

Il y a donc un intérêt croissant à utiliser les microalgues comme source alternative de ce caroténoïde.

Par exemple, les microalgues de type *Muriellopsis* sp., *Chlorella zofingiensis, Scenedesmus almeriensis* et *Chlore*/*la protothecoides* ont déjà été proposées comme des sources potentielles de lutéine.

Néanmoins, les productivités en lutéine décrites ne sont pas suffisamment élevées pour être économiquement viables à l'échelle industrielle.

Afin d'obtenir de la lutéine ou une composition enrichie en lutéine à partir des cellules d'algues cultivées, de nombreuses procédures ont été décrites, par exemple celle de la demande internationale WO 89/006910.

Dans le brevet EP 1808483, le procédé plus particulièrement mis en oeuvre consiste en la centrifugation, la sédimentation ou la filtration sous vide pour concentrer les cellules, et le séchage des cellules concentrées.

La biomasse cellulaire séchée est alors de préférence stockée à basse température (par exemple, - 20°C ou même plus bas) en emballage sous vide ou, de préférence, par l'introduction d'azote dans les sacs en plastique pour éliminer l'oxygène.

Le brevet EP 1808483 recommande également l'ajout d'antioxydants et d'agents émulsifiants à la suspension cellulaire récoltée.

Outre la récupération d'une biomasse riche en lutéine, le brevet EP 1808483 décrit la possibilité de disposer de lutéine de meilleure biodisponibilité. Le procédé préféré comprend alors la rupture des cellules collectées et leur séchage pour obtenir une lutéine ou une composition enrichie en lutéine.

L'utilisation d'un broyeur à billes standard est préconisé, dans lequel la suspension de biomasse est désintégrée en suspension dans l'eau en présence d'un anti-oxydant approprié pour empêcher l'oxydation de la lutéine.

Après séchage, on obtient un produit en poudre de type « particules de petite taille ». La poudre ainsi obtenue peut ensuite être utilisée directement dans des applications alimentaires destinées à la consommation humaine comme les compléments alimentaires, ou utilisée en mélange avec d'autres ingrédients comme la farine de poisson en aquaculture.

Dans un autre procédé, la lutéine peut être concentrée par un procédé d'extraction par solvants non polaires ou solvants supercritiques pour être formulée en compléments alimentaires ou produits pharmaceutiques.

L'extraction de la lutéine par solvants non polaires ou par fluides supercritiques a surtout été décrite dans le domaine des plantes.

Ainsi, dans le brevet US 6,106,720, il est par exemple décrit un procédé d'extraction de caroténoïdes à partir d'algues, de jus de carotte, ou de la peau de tomate, procédé comprenant l'écoulement de dioxyde de carbone supercritique présaturé avec de l'eau dans une colonne dans des conditions de pression et de température particulièrement élevées (450 à 1200 bar et 50 à 300 °C).

Dans le brevet US 4,632,837, il est décrit un procédé pour la production d'extraits concentrés de plantes fraîches d'herbes culinaires comme l'aneth, les feuilles d'estragon et les fleurs de mimosa, procédé comprenant l'extraction par CO₂ supercritique à une température de 0 à 40 °C et à une pression de 80-200 bars, et la séparation de l'extrait par de l'éther diéthylique ou du pentane à une pression de 20 à 60 bars et une température de 0 à 20°C.

Le Brevet US 4,466,923 décrit une extraction au CO₂ supercritique de lipides issus de graines de légumes, de germes de céréales et de viande des animaux par l'application simultanée de températures de 60 à 80 °C et de pressions de 700 à 1200 bar.

Le brevet US 5,120,558 décrit quant à lui un procédé d'extraction à partir d'épices telles que la sauge, la vanille, le poivre, le céleri, le gingembre, la cannelle, procédé par CO₂ supercritique opéré en continu avec quatre cuves d'extraction, entre 400 et 600 bar et une température de 80 à 120 °C, et le fractionnement des épices pour obtenir une oléorésine.

Ainsi, tous ces procédés ont surtout été mis en oeuvre pour les plantes supérieures et aucun d'entre eux ne suggère l'extrapolation possible de tels procédés d'extraction de lutéine à partir de microalgues en général, et de chlorelles en particulier.

Par ailleurs, il apparaît clairement que ces procédés souffrent de l'inconvénient selon lequel il est nécessaire d'améliorer le pouvoir solvant du fluide supercritique, soit en mettant en oeuvre des conditions opératoires de pression et de température élevées, soit en le combinant avec un autre solvant organique non polaire.

Par conséquent, le problème sous-jacent de la présente invention est de proposer un procédé alternatif d'obtention d'une composition riche en lutéine produite par des microalgues.

### RESUME DE L'INVENTION

L'invention a pour objet un procédé de préparation d'une composition riche en lutéine produite par des microalgues, plus particulièrement des microalgues de la famille des chlorelles, plus particulièrement encore produite par *Chlorella vulgaris,* caractérisé en ce qu'il comprend :
1) la préparation d'un lysat cellulaire de la biomasse de microalgues,
2) le traitement de la biomasse de microalgues lysée par un solvant polaire présentant un moment dipolaire non nul afin d'obtenir une oléorésine contenant la lutéine et les lipides de la biomasse initiale,
3) l'extraction de l'oléorésine obtenue à l'étape 2) par un fluide de dioxyde de carbone à pression supercritique, afin d'obtenir une fraction riche en lipides non polaires, principalement constituée de triglycérides, et une fraction insoluble riche en lutéine, et
4) la récupération de la fraction ainsi enrichie en lutéine.

Dans certains modes de réalisation, le solvant polaire de l'étape 2) est choisi dans le groupe constitué par les alcools tels que le méthanol, l'éthanol, le n-propanol, l'isopropanol, butanol, l'isobutanol, les esters comme l'acétate d'éthyle, l'acétate de propyle, l'acétate de butyle, les cétones comme l'acétone, la cyclohexanone, la méthyle-éthyle cétone et la méthyle-isobutyle cétone, pris seul ou en combinaison, et est de préférence l'acétate d'éthyle.

Dans un autre mode de réalisation, le procédé selon l'invention est caractérisé en ce que le fluide de dioxyde de carbone à pression supercritique est porté à une pression comprise entre 10 et 50 MPa, plus préférentiellement entre 25 et 40 MPa, et à une température comprise entre 35 et 90 °C, et préférentiellement entre 40 et 70 °C. De préférence, le solvant non polaire est le dioxyde de carbone.

A l'étape 1), le lysat cellulaire peut être obtenu par broyage d'une biomasse de microalgues de la famille des chorelles ayant une teneur en matière sèche allant de 10 à 15% en poids. Avant la mise en oeuvre de l'étape 2), le lysat cellulaire obtenu à l'étape 1) peut être concentré à une teneur en matière sèche supérieure à 15% en poids, de préférence allant de 20% à 30% en poids.

Dans certains modes de réalisation, l'étape 2) du procédé comprend une étape d'extraction du lysat cellulaire par un solvant polaire, de préférence l'acétate d'éthyle, et une étape d"élimination du solvant polaire de la phase organique obtenue. L'élimination du solvant peut être effectuée par évaporation sous vide ou à pression réduite.

L'invention a également pour objet une composition enrichie en lutéine obtenue par le procédé selon l'invention et l'utilisation de ladite composition enrichie en lutéine pour la préparation d'une composition pharmaceutique, d'un complément alimentaire ou d'un aliment.

Enfin, un objet supplémentaire selon l'invention est une méthode pour la préparation d'une composition comprenant de la lutéine choisie parmi un complément alimentaire, un aliment et une composition pharmaceutique, ledit procédé comprenant :
a) la préparation d'une fraction enrichie en lutéine à partir d'une biomasse de microalgues par la mise en oeuvre du procédé selon l'invention et
b) l'obtention de ladite composition à partir de la fraction enrichie en lutéine obtenue à l'étape a).

### FIGURES

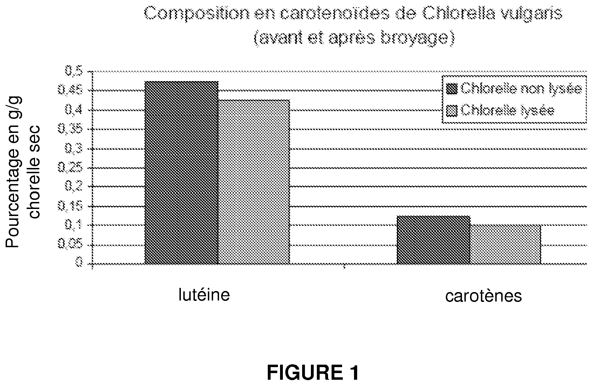

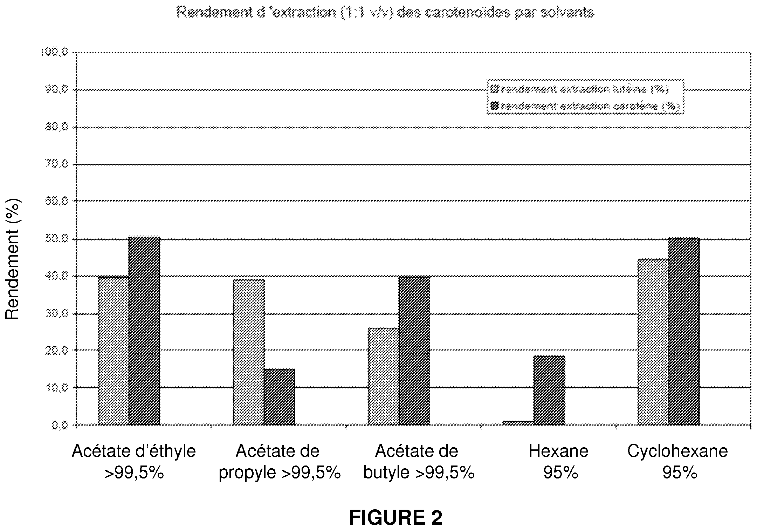

La **Figure 1** présente le dosage des caroténoïdes totaux (carotène et lutéine) de *Chlorella Vulgaris* avant broyage (diagrammes gris foncés) et après broyage (diagrammes gris clairs) de la biomasse. Les résultats sont exprimés en pourcentage massique de lutéine ou de carotène par rapport au poids sec de la biomasse.
Différents solvants d'extraction ont été testés pour la mise en oeuvre de l'étape 2) du procédé. La **Figure 2** montre les résultats d'extraction pour la lutéine (diagrammes gris clairs) et les carotènes (diagrammes gris foncés) obtenus par extraction du lysat cellulaire avec différents solvants. De gauche à droite : acétate d'éthyle, acétate de propyle, acétate de butyle, hexane et cyclohexanone. Les rendements sont exprimés en pourcentage massique de lutéine ou de carotène.

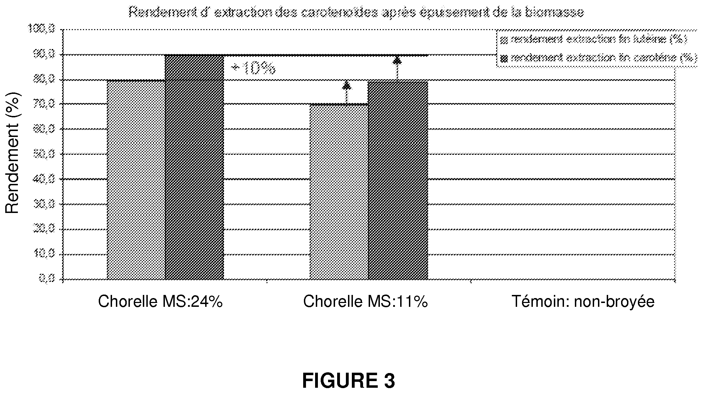

La **Figure 3** illustre l'influence de la teneur en matière séche du lysat cellulaire sur les rendements d'extraction en carotène (diagrammes gris foncés) et en lutéine (diagrammes gris clairs) obtenus à l'étape 2). De gauche à droite : lysat cellulaire de *chorelle* concentré à 24% de matière sèche (chorelle MS : 24%), lysat cellulaire de *chorelle* concentré à 11% de matière sèche (chorelle MS : 11%) et biomasse non lysée non broyée (témoin).

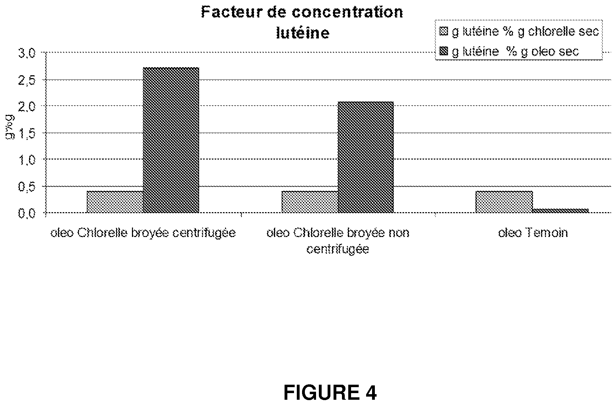

La **figure 4** qui montre les teneurs (% massique par rapport au poids sec) de lutéine dans les biomasses de départ (diagrammes gris clairs) et dans les oléorésines obtenues à partir de ces biomasses (diagrammes gris foncés). De gauche à droite : Biomasse de *chorelle* broyée et centrifugée (lysat cellulaire concentré à 24% de matière sèche (MS)), Biomasse de *chorelle* broyée, non-centrifugée (lysat cellulaire à 11% de matière sèche), Biomasse de *chorelle* non-lysée (témoin).

### DESCRIPTION DETAILLEE DE L'INVENTION

Soucieuse de mettre au point un procédé plus efficace d'obtention d'une composition riche en lutéine produite par des microalgues, plus particulièrement des microalgues de la famille des Chlorelles, plus particulièrement encore produite par *Chlorella vulgaris,* la société Demanderesse a développé ses propres recherches et a réussi à adapter les technologies d'extraction par fluide supercritique de manière à garantir un enrichissement en lutéine.

La présente invention est donc relative à un procédé de préparation d'une composition riche en lutéine produite par des microalgues, plus particulièrement des microalgues de la famille des chlorelles, plus particulièrement encore produite par *Chlorella vulgaris,* caractérisé en ce qu'il comprend la suite des étapes suivantes :
1) préparation d'un lysat cellulaire de la biomasse de microalgues,
2) traitement de la biomasse de microalgues lysée par un solvant polaire présentant un moment dipolaire non nul afin d'obtenir une oléorésine contenant la lutéine et les lipides de la biomasse initiale,
3) extraction de l'oléorésine obtenue à l'étape 2) par un fluide de dioxyde de carbone à pression supercritique, afin d'obtenir une fraction riche en lipides non polaires, principalement constituée de triglycérides, et une fraction insoluble riche en lutéine,
4) récupération de la fraction ainsi enrichie en lutéine.

Les microorganismes sont préférentiellement des microalgues appartenant à la famille des chlorelles plus préférentiellement encore *Chlorella vulgaris.*

La première étape du procédé conforme à l'invention consiste donc à préparer un lysat cellulaire de la biomasse de microalgues.

On sait classiquement cultiver les microalgues du genre *Chlorella,* et plus particulièrement *Chlorella vulgaris* dans des photobioréacteurs clos, généralement tubulaires, où l'on peut injecter du gaz carbonique en haute concentration.

Ces cultures en conditions autotrophes permettent classiquement d'obtenir une concentration en microalgues *Chlorella vulgaris* de l'ordre de 50 à 80 g/l.

La récupération et concentration de la biomasse s'effectuent ensuite par tous moyens connus par ailleurs de l'homme du métier, telle que la centrifugation.

Dans le cadre de l'invention, la biomasse de microalgues ainsi collectée puis concentrée peut présenter une matière sèche comprise entre 10 et 15 %, de préférence une matière sèche de l'ordre de 11 %.

Pour extraire les molécules d'intérêt, dont les caroténoïdes, une étape de broyage cellulaire (c'est-à-dire de préparation d'un lysat cellulaire) est souvent indispensable.

Pour extraire les molécules d'intérêt dans des conditions non dénaturantes, il est préférable de conduire le broyage cellulaire, le plus possible à froid, sous atmosphère inerte et à l'abri de la lumière.

L'augmentation de la température et la lumière sont en effet susceptibles d'initier l'oxydation des molécules.

Ainsi, dans certains modes de réalisation du procédé selon l'invention, l'étape 1 comprend la préparation d'un lysat cellulaire par broyage à partir d'une biomasse de microalgues. De préférence, la biomasse de microalgues a une teneur en matière sèche allant de 10 à 15% en poids par rapport à son poids total. Dans un autre mode de réalisation préféré, l'étape de broyage est réalisée à froid, à l'abri de la lumière et sous atmosphère inerte.

La société Demanderesse recommande d'utiliser la technologie de broyage à billes, en mode recirculation ou passage comme il sera exemplifié ci-après. Toutefois afin d'accroitre les rendements d'extraction des molécules d'intérêt, le broyage peut être envisagé sur une biomasse de microalgues concentrée et le broyage réalisé en phase solvant.

L'efficacité du broyage est suivie sous microscope optique (grossissement X40) ; la lyse étant totale lorsque plus aucune cellule intacte n'est visible dans le champ du microscope.

Comme il sera exemplifié ci-après, cette étape de broyage n'engendre qu'une perte d'au plus 10 % en caroténoïdes totaux.

La seconde étape du procédé conforme à l'invention consiste à traiter la biomasse de microalgues lysée par un solvant polaire afin d'obtenir une oléorésine contenant la lutéine et les lipides de la biomasse initiale.

On entend par « solvant polaire » tout solvant présentant un moment dipolaire non nul.

Le solvant polaire est choisi dans le groupe constitué du méthanol, de l'éthanol, du n-propanol et de l'isopropanol, du butanol et de l'isobutanol, des esters comme l'acétate d'éthyle, l'acétate de propyle, l'acétate de butyle, des cétones comme l'acétone, la cyclohexanone, la méthyle-éthyle cétone et la méthyle-isobutyle cétone, pris seul ou en combinaison, et est de préférence un ester, et de manière encore plus préférée l'acétate d'éthyle.

La société Demanderesse recommande d'utiliser l'acétate d'éthyle comme solvant d'extraction car, comme il sera exemplifié ci-après, de tous les solvants testés, il se révèle le plus efficace et est par ailleurs relativement peu toxique.

Le solvant d'extraction peut être éliminé de la fraction organique, de préférence par évaporation, par exemple sous vide ou à pression réduite, ce par quoi l'oléorésine est obtenue.

Comme il sera également exemplifié ci-après, l'extraction répétée à l'acétate d'éthyle, par épuisement de la biomasse de *Chlorella vulgaris* à 11 % de matière sèche, préparée à l'étape précédente du procédé conforme à l'invention, permet de concentrer la lutéine d'un facteur 5 à 10 et d'atteindre des rendements d'extraction de l'ordre de 70 %.

Ainsi, dans certains modes de réalisation du procédé selon l'invention, l'étape 2 comprend au moins une étape d'extraction du lysat cellulaire par un solvant polaire, de préférence l'acétate d'éthyle, ladite étape d'extraction pouvant être répétée. L'étape d'extraction peut être répétée jusqu'à l'obtention d'un rendement en lutéine d'au moins 50%, de préférence d'au moins 60%, voire d'au moins 65% en poids par rapport au poids de lutéine initialement présent dans le lysat cellulaire obtenu à l'étape 1. L'étape d'extraction peut ainsi être répétée de 2 à 8 fois, typiquement 5 fois.

Le rapport volumique solvant polaire/lysat cellulaire à chaque étape d'extraction peut varier de 1:3 à 3:1. Par exemple, on peut utiliser un rapport volumique d'environ 1:1.

Les fractions organiques (surnageants) peuvent être regroupées et le solvant polaire peut être éliminé, au moins partiellement, par évaporation, de préférence sous vide ou à pression, conduisant ainsi à l'obtention de l'oléorésine.

De manière facultative, puisque l'eau est un facteur limitant, la société Demanderesse recommande également d'ajouter une étape de concentration du lysat cellulaire (par exemple par centrifugation) avant de réaliser l'extraction avec le solvant polaire, car cela permet d'augmenter le rendement global d'extraction d'au moins 10 %. L'extraction peut également être envisagée à partir d'une matrice déshydratée.

La concentration de la biomasse lysée à 24 % de matière sèche permet ainsi d'obtenir un rendement d'extraction de 80 %.

Ainsi, dans certains modes de réalisation du procédé selon l'invention, le lysat cellulaire obtenu à l'étape 1 est concentré, de préférence à une teneur en matière sèche supérieure à 15% en poids, de préférence allant de 20% à 30% en poids, avant la mise en oeuvre de l'étape 2 de préparation de l'oléorésine. Typiquement, le lysat cellulaire peut être concentré à une teneur en matière sèche d'environ 22% à 26%, par exemple 24%. Cette étape de concentration peut être effectuée par centrifugation.

La troisième étape du procédé conforme à l'invention consiste à extraire l'oléorésine obtenue à l'étape 2) par un solvant non polaire, ici un fluide à pression supercritique, afin d'obtenir une fraction riche en lipides non polaires, principalement constituée de triglycérides, et une fraction insoluble riche en lutéine.

La fraction insoluble riche en lutéine, autrement dit la composition riche en lutéine, correspond à la fraction d'oléorésine qui n'a pas été entraînée par le fluide à pression supercritique.

La fraction riche en lipides non polaires, principalement constituée de triglycérides, correspond à la fraction qui est entrainée par le solvant non polaire à pression supercritique et qui y est donc soluble dans les conditions de température et de pression utilisée dans ledit solvant.

La troisième étape du procédé permet l'extraction sélective des lipides non polaires, l'extrait étant ensuite fractionné par évaporation du solvant qui est condensé et recyclé, avec récupération d'une pâte (appelée communément une "concrète") riche en lipides non polaires, principalement constitués de triglycérides ; le résidu insoluble dans le fluide à pression supercritique est riche en lutéine qui est ainsi fortement concentrée et récupérée avec un rendement élevé par rapport à la biomasse initiale, après élimination du fluide à pression supercritique présent de façon résiduelle dans ce résidu.

On sait qu'un fluide en état supercritique, c'est-à-dire dans un état caractérisé soit par une pression et une température respectivement supérieures à la pression et à la température critiques dans le cas d'un corps pur, soit par un point représentatif (pression, température) situé au-delà de l'enveloppe des points critiques représentés sur un diagramme (pression, température) dans le cas d'un mélange, présente, pour de très nombreuses substances, un pouvoir solvant élevé sans commune mesure avec celui observé dans ce même fluide à l'état de gaz comprimé ; il en est de même des liquides dits « subcritiques », c'est-à-dire dans un état caractérisé soit par une pression supérieure à la pression critique et par une température inférieure à la température critique dans le cas d'un corps pur, soit par une pression supérieure aux pressions critiques et une température inférieure aux températures critiques des composants dans le cas d'un mélange.

Par commodité de langage, l'usage fait que l'on appelle fluide comprimé tout fluide porté à une pression sensiblement supérieure à la pression atmosphérique ; on appelle fluide à pression supercritique un fluide porté à une pression supérieure à sa pression critique, c'est-à-dire soit un fluide supercritique proprement dit, soit un liquide dit sub-critique comme défini ci-dessus ; de même, on appelle gaz liquéfié un liquide, constitué d'un composé qui se trouve à l'état gazeux à pression atmosphérique et à température ambiante, porté à une pression et à une température inférieures à sa pression et sa température critiques respectivement.

Les variations importantes et modulables du pouvoir solvant des fluides à pression supercritique sont utilisées dans de nombreux procédés d'extraction (solide/fluide), de fractionnement (liquide/fluide), de chromatographie analytique ou préparative, de traitement des matériaux (céramiques, polymères, ...) ; des réactions chimiques ou biochimiques sont également réalisées dans de tels solvants.

Il est à noté que les propriétés physico-chimiques du dioxyde de carbone ainsi que ses coordonnées critiques (pression critique : 7,4 MPa et température critique : 31 °C) en font le solvant préféré dans de nombreuses applications, surtout qu'il ne présente pas de toxicité et est disponible à très bas prix en très grande quantité ; solvant non polaire, le dioxyde de carbone porté à pression supercritique est parfois additionné d'un co-solvant consistant en un solvant organique polaire qui va modifier le pouvoir solvant de façon notable surtout vis-à-vis de molécules présentant une certaine polarité, l'éthanol étant souvent utilisé à cette fin.

Toutefois, certains composés sont plus favorablement extraits par un hydrocarbure léger ayant entre 2 et 5 atomes de carbone, et plus favorablement, entre 2 et 4 atomes de carbone, à pression supercritique.

Comme il est connu de l'homme du métier, l'extraction par un fluide à pression supercritique conduit à des extraits de très grande qualité qui sont de plus en plus utilisés dans de nombreuses applications.

L'un des avantages principaux des procédés mettant en oeuvre les fluides à pression supercritique réside dans la facilité de réaliser la séparation entre le solvant (le fluide) et les extraits et solutés.

L'un des autres avantages importants des fluides supercritiques réside dans leur sélectivité « adaptable » vis-à-vis des composants d'un mélange. Cette très haute sélectivité est liée aux propriétés particulières des fluides supercritiques, et particulièrement à celles du dioxyde de carbone à pression supercritique : Le pouvoir solvant peut être finement réglé en jouant sur la pression et la température du fluide ; il a été trouvé que des conditions « douces » sont les plus sélectives car le solvant est d'autant plus sélectif que son pouvoir solvant est plus faible.

Ainsi, dans certains modes de réalisation du procédé selon l'invention, à l'étape 3), le fluide de dioxyde de carbone à pression supercritique est porté à une pression comprise entre 10 et 50 MPa, plus préférentiellement entre 25 et 40 MPa, et à une température comprise entre 35 et 90 °C, et préférentiellement entre 40 et 70 °C. Dans d'autres modes de réalisation, à l'étape 3), le solvant non polaire sous forme d'un fluide à pression supercritique est le dioxyde de carbone.

La société Demanderesse utilise, de préférence, pour la mise en oeuvre de l'étape 3), le dioxyde de carbone pur, plutôt qu'additionné d'un co-solvant qui augmenterait son pouvoir solvant, et choisit une pression d'opération comprise entre 10 et 50 MPa, plus préférentiellement entre 25 et 40 MPa, et à une température comprise entre 35 et 90 °C, et préférentiellement entre 40 et 70 °C.

A titre d'exemple, l'étape 3) peut être mise en oeuvre à l'aide de CO₂ à une pression de 25 à 30 MPa, typiquement environ 28 MPa, et à une température de 40 °C à 50 °C, typiquement environ 45°C.

C'est ainsi que la société Demanderesse a trouvé qu'il est possible d'extraire sélectivement les lipides non polaires, de type triglycérides, d'une concrète obtenue lors de l'étape 3 du procédé conforme à l'invention sans extraite la lutéine qui va ainsi se trouver fortement concentrée dans le raffinat non extrait.

Dans un mode de réalisation particulier, le procédé selon l'invention comprend une ou plusieurs (1, 2, 3, 4, 5 ou 6) des caractéristiques suivantes :
- Les microalgues sont du genre *Chlorella,* et plus particulièrement de l'espèce *Chlorella vulgaris,* et/ou
- l'étape 1) comprend la préparation d'un lysat cellulaire par broyage à partir d'une biomasse de microalgues ayant une teneur en matière sèche allant de 10 à 15%, typiquement environ 11% en poids, et/ou
- le lysat cellulaire obtenu à l'étape 1 est concentré de manière à avoir une teneur en matière sèche supérieure à 15% en poids, de préférence allant de 20% à 30% en poids, et/ou
- l'étape 2) comprend au moins une étape d'extraction du lysat cellulaire par un solvant polaire, de préférence l'acétate d'éthyle, et l'élimination du solvant polaire de la ou des phases organiques obtenues de manière à obtenir l'oléorésine, et/ou
- dans l'étape 2), l'étape d'extraction est répétée jusqu'à l'obtention d'un rendement d'extraction en lutéine d'au moins 50% en poids par rapport au poids de lutéine contenu initialement dans le lysat cellulaire, et/ou
- à l'étape 3), l'oléorésine obtenue à l'étape 2) est extraite par du CO₂ à pression supercritique, de préférence à une pression entre 10 et 50 MPa, de manière plus préférée entre 25 et 40 MPa et à une température entre 35 et 90 °C, de préférence entre 40 °C et 70 °C.

Il va de soi que le procédé selon l'invention peut comprendre une ou plusieurs étapes supplémentaires à celles précédemment citées, par exemple une étape de conditionnement de la composition enrichie en lutéine obtenue à l'étape c).

L'invention a également pour objet une méthode de préparation d'une composition comprenant de la lutéine choisie parmi un complément alimentaire et une composition pharmaceutique, ladite méthode comprenant :
a) la préparation d'une fraction enrichie en lutéine à partir d'une biomasse de microalgues par la mise en oeuvre du procédé selon l'invention et
b) l'obtention de ladite composition à partir de la fraction enrichie en lutéine obtenue à l'étape a).

L'étape b) comprend généralement le mélange de la fraction enrichie en lutéine avec un ou plusieurs excipients ou véhicules acceptables sur le plan alimentaire et/ou pharmaceutique de manière à obtenir le complément alimentaire ou la composition pharmaceutique souhaité(e). Le complément alimentaire ou la composition pharmaceutique peuvent se présenter sous la forme d'une poudre, d'un comprimé, d'une suspension, d'un sirop ou encore d'une solution buvable.

La fraction enrichie en lutéine peut également être utilisée pour la préparation d'un aliment destiné à l'alimentation humaine ou animale. L'invention a également pour objet une méthode de préparation d'un aliment, ladite méthode comprenant :
a) la préparation d'une fraction enrichie en lutéine à partir d'une biomasse de microalgues par la mise en oeuvre du procédé selon l'invention et
b) l'obtention dudit aliment à partir de la fraction enrichie en lutéine obtenue à l'étape a).

L'étape b) comprend généralement le mélange de la composition enrichie en lutéine avec un ou plusieurs ingrédients alimentaires ainsi que des étapes telles que des étapes de cuisson ou de refroidissement.

L'invention sera mieux comprise à l'aide des exemples qui suivent, lesquels se veulent illustratifs et non limitatifs.

### Exemple 1 : Préparation d'une oléorésine concentrée en lutéine à partir d'une biomasse de Chlorella vulgaris

### # Etape 1 : Préparation d'une biomasse de Chlorelle vulgaris d'une matière sèche de 11 à 12 %

Par fermentation classique en photobioréacteur, il est aisé d'obtenir en fin de fermentation une biomasse présentant une concentration en microalgues de l'ordre de 78 g/l. Celle-ci est alors concentrée par centrifugation sur centrifugeuse de type WESTFALIA modèle NA7. La biomasse est ainsi concentrée à environ 120 g/l.

### # Etape 2 : Broyage cellulaire

La biomasse de microalgues ainsi cultivée puis concentrée selon les conditions opératoires de l'étape 1 est alors traitée par un système de broyeur agitateur à billes LABSTAR de NETZSCH.

6 litres de biomasse à 12 % de matière sèche récupérés lors de l'étape 1 ont été traités en mode recirculation.

Le produit subit donc un passage répété dans la chambre de broyage de 0,54 litres remplie à 85% par des billes en céramique type *Zeta beads* (NETZSCH) de 600 µm de diamètre. Ces billes sont mises en agitation à une vitesse périphérique de 12 m/s, ce qui permet de broyer la quasi-totalité des cellules de microalgues en 2 h. La qualité du broyage est suivie par observation microscopique jusqu'à ce qu'aucune cellule ne soit visible dans le champ du microscope.

Afin de limiter l'augmentation de température du produit, la chambre de broyage est dotée d'une double enveloppe permettant un maintien à froid. La cuve d'alimentation du broyeur est également refroidie par une recirculation d'eau froide à 4°C fournie par un cryostat Julabo de type F32.

Les résultats de dosage de caroténoïdes totaux obtenus lors de cette étape de broyage cellulaire sont présentés dans la figure 1. La **Figure 1** présente le dosage des caroténoïdes totaux (carotène et lutéine) de *Chlorella Vulgaris* avant et après broyage (% massique par rapport au poids sec). Le broyage cellulaire engendre une perte raisonnable de 10 % en lutéine et 20 % en carotènes.

### # Etape 3 : Extraction par solvant

Le broyat de microalgues obtenu à la suite de l'étape 2, est alors dilué avec des solvants de différentes polarités comme des esters dont l'acétate d'éthyle, l'acétate de propyle, l'acétate de butyle, des cétones comme la cyclohexanone et un solvant apolaire comme l'hexane. Chaque solvant a été testé seul, dans un ratio volumique de 1 :1 (V biomasse/ V solvant).

L'extraction est alors réalisée sous agitation, dans l'obscurité et à température ambiante pendant une heure, l'ensemble de la manipulation est réalisée dans des tubes en polypropylène.

Après 1 heure de contact, le mélange est séparé par centrifugation 15 min à 20000 g sur centrifugeuse BECKMAN Coulter Allegra 64R. Les dosages en caroténoïdes ont été réalisées sur le surnageant de chaque solvant testé après une seul extraction.

Les résultats de rendement d'extraction sont présentés dans la figure 2.

La **Figure 2** présente le rendement d'extraction des caroténoïdes (lutéine et carotène) totaux pour différents solvants: acétate d'éthyle (pureté >99,5%), acétate de propyle (pureté > 99,5%), hexane (pureté > 95%) et cyclohexane (pureté > 95%). Ordonnées : pourcentage d'extraction.

La meilleure extraction en lutéine est obtenue avec les acétates d'éthyle, de propyle et la cyclohexanone. Ces essais réalisés en une seule extraction permettent la récupération d'environ 40 % de lutéine.

Contrairement à l'acétate de propyle, la cyclohexanone et l'acétate d'éthyle ont de très bons rendements d'extraction pour les carotènes.

Ce screening démontre également que plus la chaîne aliphatique de l'ester est longue plus le rendement d'extraction de la lutéine tend à diminuer, ce résultat oriente donc vers les caractéristiques du solvant à utiliser.

Bien que l'acétate éthyle et le cyclohexanone présentent des rendements d'extraction très proches, l'acétate d'éthyle est préféré puisque ce solvant est tout aussi efficace que la cyclohexanone mais est surtout moins toxique.

### # Etape 4 : Concentration d'un broyat cellulaire

Une partie du broyat cellulaire obtenu à l'étape 2 est concentré par centrifugation sur BECKMAN Coulter J20XP Rotor JLA à 6000 g pendant 3 min.

La préparation de l'oléorésine à été réalisée sur 3 échantillons différents :
- biomasse broyée puis concentrée par centrifugation (24 % de matière sèche)
- biomasse broyée non concentrée (11% en matière sèche)
- un témoin non broyé, non concentré. (11% en matière sèche)

### # Etape 5: Préparation de l'oléorésine - Extraction à l'acétate d'éthyle par épuisement

Dans un flacon en polypropylène de 1 litre résistant aux produits chimique, un ratio volumique de 1 pour 1 de biomasse broyée et d'acétate d'éthyle *(pureté mini 99,8* %) sont mélangé.

Ce mélange est ensuite agité, à l'abri de la lumière et à température ambiante. Après 1 heure de contact le mélange est séparé par centrifugation sur BECKMAN Coulter J20XP Rotor JLA pendant 15 min à 12227 g.

Le surnageant ainsi obtenu (environ 0,4 litre) est réservé dans un flacon à l'obscurité. Quant au culot, il est à nouveau repris par du solvant. Le volume de reprise est identique au volume précédemment aliquoté.

Cette opération est réalisée 5 fois sur le même culot de biomasse. Toujours à l'abri de la lumière, les 5 surnageants ainsi obtenus sont ensuite regroupés en une fraction (F) qui est analysée.

La **figure 3** présente les rendements d'extraction en lutéine et en carotène par l'acétate d'éthyle des 3 fractions obtenues lors de l'extraction par épuisement des biomasses préparées à l'étape 4 c'est-à-dire des biomasses broyées (ayant une matière sèche (MS) de 24% et de 11%) et de la biomasse non-broyée

Les meilleurs rendements d'extraction atteignent 80% de récupération de lutéine et 90% de caroténoïdes pour l'échantillon de biomasse à 24% de MS.

Après une extraction, sont déjà obtenus 40% de récupération de lutéine (1,3), mais après 5 extractions successives le rendement atteint 70%.

Ce graphique montre également que les rendements sont 10 % plus élevés sur la biomasse concentrée (MS à 24%) que sur la biomasse initiale (MS à 11%). Le rendement d'extraction est donc limité par la présence d'eau.

L'absence totale de lutéine et de carotène sur le témoin non broyé, démontre l'importance de la lyse cellulaire pour la récupération des molécules d'intérêt.

### # Etape 6: Préparation de l'oléorésine - Concentration des Caroténoïdes totaux

Les différentes fractions F préparées à l'étape 5, sont alors transférées dans un ballon ambré de 2 litres qui est connecté à un évaporateur rotatif sous vide de marque Buchi Switzerland de type Rotavapor R-215.

L'évaporation du solvant se déroule alors pendant 2h à un vide de 200 mbar et à 50 °C.

Les différents résultats obtenus sont compilés dans la **figure 4** qui montre les teneurs (% massique par rapport au poids sec) de lutéine dans les biomasses et les oléorésines.

Les matières sèches finales d'oléorésine sont de 65 à 80 % et la quantité de lutéine voisine de 2 à 3 g pour 100 g d'oléorésine sèche.

L'extraction d'une oléorésine à partir d'un broyat de *Chlorelle Vulgaris* concentré à 24 % MS, permet une concentration par 7 de la lutéine.

### Exemple 2. Extraction d'une oléorésine riche en lutéine par fluide supercritique

### • Etape 1 : préparation du lysat cellulaire

La biomasse est sous forme d'une suspension aqueuse de *Chlorella vulgaris* lysée selon les conditions de l'exemple 1. La mesure de la matière sèche et l'analyse quantitative des pigments sont présentées dans le tableau 1 ci-dessous.

**Tableau 1**

| | Méthode | Quantification |
|---|---|---|
| Matière sèche | Pesée sur dessicateur | 12,1% matière sèche |
| Caroténoïdes totaux | Spectrophotométrie UV | 9,9 mg / g mat. sèche |
| Chlorophylle A | Spectrophotométrie UV | 19,01 mg / g mat. sèche |
| Chlorophylle B | Spectrophotométrie UV | 8,93 mg / g mat. sèche |
| Lutéine | HPLC | 2,89 mg / g mat. sèche |
| Carotènes | HPLC | 0,30 mg / g mat. sèche |

### • Etape 2 : Obtention de l'oléorésine

La biomasse lysée est mélangée avec de l'acétate d'éthyle à raison de 1 050 g de biomasse, contenant 1 260 mg de caroténoïdes totaux dont 368 mg de lutéine, et 500 g d'acétate d'éthyle dans une fiole de 3 litres dotée d'un agitateur mécanique pendant deux heures. Le mélange ainsi obtenu est décanté par centrifugation : Le surnageant est récupéré et le culot est soumis à une seconde extraction.

Cette seconde extraction est mise en oeuvre dans les mêmes conditions que précédemment en utilisant à nouveau 500 g d'acétate d'éthyle. Le mélange obtenu est décanté par centrifugation : Le surnageant est récupéré et le culot est soumis à une troisième extraction dans les mêmes conditions que la seconde extraction.

Et ainsi de suite jusqu'à cinq opérations d'extraction et la mise en oeuvre de 2 500 g d'acétate d'éthyle.

Les surnageants ainsi recueillis sont regroupés en une solution qui est alors évaporée dans un évaporateur rotatif sous vide, conduisant à récupérer le solvant d'une part et une oléorésine d'autre part.

La masse d'oléorésine ainsi obtenue est de 11,95 g et contient 550 mg de caroténoïdes totaux dont 310 mg de lutéine.

### • Etape 3 : Extraction par fluide supercritique

L'oléorésine obtenue lors de l'étape précédente est introduite dans un panier cylindrique fermé par deux filtres en métal fritté qui est lui-même installé dans un récipient sous pression, raccordé à une pompe alimentée en CO₂ liquide débitant 3 kg/h, le fluide ainsi comprimé à 28 MPa étant ensuite réchauffé à 45 °C et introduit dans le récipient sous pression contenant le panier chargé de l'oléorésine.

Le fluide sortant du récipient sous pression après s'être chargé en soluté au contact de l'oléorésine est ensuite détendu à 5 MPa au sein de deux séparateurs cycloniques maintenus à 45 °C : Les solutés sont séparés et récupérés dans les séparateurs et le CO₂ est rejeté à l'atmosphère.

Après deux heures d'extraction, le récipient sous pression est dépressurisé, ouvert et le panier est récupéré. Le résidu contenu dans le panier se présente sous forme d'une poudre sèche d'une masse de 1,75 g contenant 218 mg de lutéine, soit une teneur massique en lutéine de 12% par rapport au poids total du résidu.

## Revendications

1. Procédé de préparation d'une composition riche en lutéine produite par des microalgues **caractérisé en ce qu'**il comprend :
1) la préparation d'un lysat cellulaire de la biomasse de microalgues,
2) le traitement de la biomasse de microalgues lysée par un solvant polaire présentant un moment dipolaire non nul afin d'obtenir une oléorésine contenant la lutéine et les lipides de la biomasse initiale,
3) l'extraction de l'oléorésine obtenue à l'étape 2) par un fluide de dioxyde de carbone à pression supercritique, afin d'obtenir une fraction riche en lipides non polaires, principalement constituée de triglycérides, et une fraction insoluble riche en lutéine, et
4) la récupération de la fraction ainsi enrichie en lutéine.

2. Procédé selon la revendication 1, **caractérisé en ce que** le solvant polaire de l'étape 2) est choisi dans le groupe constitué par le méthanol, l'éthanol, le n-propanol et l'isopropanol, le butanol et l'isobutanol, les esters comme l'acétate d'éthyle, l'acétate de propyle, l'acétate de butyle, des cétones comme l'acétone, la cyclohexanone, la méthyle-éthyle cétone et la méthyle-isobutyle cétone, pris seul ou en combinaison, et est de préférence l'acétate d'éthyle.

3. Procédé selon l'une ou l'autre des revendications 1 et 2, **caractérisé en ce que** le fluide de CO₂ à pression supercritique est porté à une pression comprise entre 10 et 50 MPa et à une température comprise entre 35 et 90°C.

4. Procédé selon l'une quelconque des revendications 1 à 3, **caractérisé en ce que** le fluide de CO₂ à pression supercritique est porté à une pression comprise entre 25 et 40 MPa, et à une température comprise entre 40 et 70°C.

5. Procédé selon l'une quelconque des revendications précédentes **caractérisé en ce qu'**à l'étape 1, le lysat cellulaire est obtenu par broyage d'une biomasse de microalgues de la famille des chorelles ayant une teneur en matière sèche allant de 10 à 15% en poids.

6. Procédé selon l'une quelconque des revendications précédentes, dans lequel le lysat cellulaire obtenu à l'étape 1) est concentré à une teneur en matière sèche supérieure à 15% en poids, de préférence allant de 20% à 30% en poids avant la mise en oeuvre de l'étape 2).

7. Procédé selon l'une quelconque des revendications précédentes dans lequel l'étape 2) comprend au moins une étape d'extraction du lysat cellulaire par un solvant polaire, de préférence l'acétate d'éthyle, et l'élimination du solvant polaire de la phase organique obtenue.

8. Procédé selon l'une quelconque des revendications précédentes dans lequel les microalgues appartiennent à la famille des chlorelles.

9. Procédé selon l'une quelconque des revendications précédentes dans lequel la lutéine est produite par *Chlorella vulgaris.*

10. Méthode pour la préparation d'une composition comprenant de la lutéine choisie parmi un complément alimentaire, un aliment, et une composition pharmaceutique, ladite méthode comprenant :
a) la préparation d'une fraction enrichie en lutéine à partir d'une biomasse de microalgues par la mise en oeuvre du procédé selon l'une quelconque des revendications 1 à 9 et
b) l'obtention de ladite composition à partir de la fraction enrichie en lutéine obtenue à l'étape a).

## Patentansprüche

1. Verfahren zur Herstellung einer Zusammensetzung, die reich an von Mikroalgen produziertem Lutein ist, **dadurch gekennzeichnet, dass** es umfasst:
1) Herstellung eines Zelllysats der Mikroalgenbiomasse,
2) Behandlung der lysierten Mikroalgenbiomasse mit einem polaren Lösemittel, das ein Dipolmoment ungleich null aufweist, um ein Oleoresin zu erhalten, das Lutein und Fette der Ausgangsbiomasse enthält,
3) Extraktion des in Schritt 2) erhaltenen Oleoresins mit einem Kohlenstoffdioxid-Fluid mit überkritischem Druck, um eine Fraktion, die reich an unpolaren Lipiden ist und im Wesentlichen aus Triglyceriden besteht, und eine Lutein-reiche unlösliche Fraktion zu erhalten, und
4) Wiedergewinnung der auf diese Weise Lutein-angereicherten Fraktion.

2. Verfahren gemäß Anspruch 1, **dadurch gekennzeichnet, dass** das polare Lösemittel von Schritt 2) aus der Gruppe ausgewählt ist, bestehend aus Methanol, Ethanol, n-Propanol und Isopropanol, Butanol und Isobutanol, Estern wie Ethylacetat, Propylacetat, Butylacetat, Ketonen wie Aceton, Cyclohexanon, Methylethylketon und Methylisobutylketon, allein oder in Kombination verwendet, und vorzugsweise Ethylacetat ist.

3. Verfahren gemäß einem der beiden Ansprüche 1 und 2, **dadurch gekennzeichnet, dass** das CO₂-Fluid mit überkritischem Druck auf einen Druck zwischen 10 und 50 MPa und auf eine Temperatur zwischen 35 und 90°C gebracht wird.

4. Verfahren gemäß einem der Ansprüche 1 bis 3, **dadurch gekennzeichnet, dass** das CO₂-Fluid mit überkritischem Druck auf einen Druck zwischen 25 und 40 MPa und auf eine Temperatur zwischen 40 und 70°C gebracht wird.

5. Verfahren gemäß einem der vorhergehenden Ansprüche, **dadurch gekennzeichnet, dass** in Schritt 1) das Zelllysat durch Aufbrechen einer Biomasse von Mikroalgen der Familie Chlorellaceae mit einem Trockenmassegehalt von 10 bis 15 Gew.-% erhalten wird.

6. Verfahren gemäß einem der vorhergehenden Ansprüche, wobei das in Schritt 1) erhaltene Zelllysat auf einen Trockenmassegehalt größer als 15 Gew.-%, vorzugsweise auf 20 bis 30 Gew.-%, vor Durchführung von Schritt 2) konzentriert wird.

7. Verfahren gemäß einem der vorhergehenden Ansprüche, wobei Schritt 2) wenigstens einen Schritt der Extraktion des Zelllysats mit einem polaren Lösemittel, vorzugsweise Ethylacetat, und die Entfernung des polaren Lösemittels aus der organischen Phase umfasst.

8. Verfahren gemäß einem der vorhergehenden Ansprüche, wobei die Mikroalgen zur Familie Chlorellaceae gehören.

9. Verfahren gemäß einem der vorhergehenden Ansprüche, wobei das Lutein von *Chlorella vulgaris* produziert wird.

10. Verfahren zur Herstellung einer Lutein umfassenden Zusammensetzung, ausgewählt aus einem Nahrungsergänzungsmittel, einem Nahrungsmittel und einer pharmazeutischen Zusammensetzung, wobei besagtes Verfahren umfasst:
a) Herstellung einer Lutein-angereicherten Fraktion aus einer Mikroalgenbiomasse mittels Durchführung des Verfahrens gemäß einem der Ansprüche 1 bis 9, und
b) Erhalt besagter Zusammensetzung aus der Lutein-angereicherten Fraktion, die in Schritt a) erhalten wird.

## Claims

1. A method for preparing a composition rich in lutein produced by microalgae, **characterized in that** it comprises:
1) preparing a cell lysate from the microalgal biomass,
2) treating the lysed microalgal biomass with a polar solvent having a non-zero dipole moment in order to obtain an oleoresin containing the lutein and the lipids from the initial biomass,
3) extracting the oleoresin obtained in step 2) by a carbon dioxide fluid at supercritical pressure, in order to obtain a fraction rich in nonpolar lipids, mainly consisting of triglycerides, and an insoluble fraction rich in lutein, and
4) recovering the thus lutein-enriched fraction.

2. The method as claimed in claim 1, **characterized in that** the polar solvent of step 2) is chosen from the group made up of methanol, ethanol, n-propanol and isopropanol, butanol and isobutanol, esters such as ethyl acetate, propyl acetate or butyl acetate, and ketones such as acetone, cyclohexanone, methyl ethyl ketone and methyl isobutyl ketone, taken alone or in combination, and is preferably ethyl acetate.

3. The method as claimed in either of claims 1 and 2, **characterized in that** the carbon dioxide fluid at supercritical pressure is brought to a pressure of between 10 and 50 MPa, and to a temperature of between 35 and 90°C.

4. The method as claimed in any one of claims 1 to 3, **characterized in that** the carbon dioxide fluid at supercritical pressure is brought to a pressure of between 25 and 40 MPa, and to a temperature of between 40 and 70°C.

5. The method as claimed in any one of the preceding claims, **characterized in that**, in step 1, the cell lysate is obtained by milling a biomass of microalgae of the Chlorella family having a dry matter content ranging from 10% to 15% by weight.

6. The method as claimed in any one of the preceding claims, wherein the cell lysate obtained in step 1) is concentrated to a dry matter content of greater than 15% by weight, preferably ranging from 20% to 30% by weight, before step 2) is carried out.

7. The method as claimed in any one of the preceding claims, wherein step 2) comprises at least one step of extracting the cell lysate with a polar solvent, preferably ethyl acetate, and removing the polar solvent from the organic phase obtained.

8. The method as claimed in any one of the preceding claims, wherein the microalga belongs to the Chlorella family.

9. The method as claimed in any one of the preceding claims, wherein the lutein is produced by *Chlorella vulgaris.*

10. A method for preparing a composition comprising lutein chosen from a food supplement, a food and a pharmaceutical composition, said method comprising:
a) preparing a lutein-enriched fraction from a microalgal biomass by carrying out the method as claimed in any one of claims 1 to 9, and
b) obtaining said composition from the lutein-enriched fraction obtained in step a).
